# EUROPÄISCHE PATENTANMELDUNG

(11) **EP 4 201 347 A1**
(43) Veröffentlichungstag der Anmeldung: **28.06.2023**
(21) Anmeldenummer: 22214953.6
(22) Anmeldetag: 20.12.2022
(51) Int. Cl.: A61B 17/28, A61B 17/29

(54) **MEDIZINISCHES INSTRUMENT**

(30) Priorität: 23.12.2021 DE 102021134467
(71) Anmelder: Aesculap AG, 78532 Tuttlingen (DE)
(72) Erfinder: Pleil, Thomas, 78073 Bad Dürrheim (DE); Hauser, Josef, 78594 Gunningen (DE); Wäschle, Tobias, 78598 Königsheim (DE)
(74) Vertreter: Hoeger, Stellrecht & Partner Patentanwälte mbB

(57) **Zusammenfassung**

Um ein medizinisches Instrument mit zwei relativ zueinander bewegbaren Branchen, wobei jede Branche ein distales Ende mit einem Werkzeugelement und ein proximales Ende aufweist und wobei die Werkzeugelemente miteinander zusammenwirkend angeordnet und ausgebildet sind und eine medizinische Werkzeugeinrichtung bilden, wobei das medizinische Instrument ein vorspannendes Element umfasst, welches mit den zwei Branchen zusammenwirkend angeordnet oder ausgebildet ist, so zu verbessern, dass es eine anwenderfreundlichere Handhabung ermöglicht, wird vorgeschlagen, dass das vorspannende Element von einer Zugstellung, in welcher es eine Zugkraft auf die zwei Branchen ausübt, in eine Druckstellung, in welcher es eine Druckkraft auf die zwei Branchen ausübt, bringbar ist oder umgekehrt durch Änderung seiner Position und/oder seiner Orientierung und/oder seiner Form relativ zu den zwei Branchen.

## Beschreibung

Die vorliegende Erfindung betrifft ein medizinisches Instrument mit zwei relativ zueinander bewegbaren Branchen, wobei jede Branche ein distales Ende mit einem Werkzeugelement und ein proximales Ende aufweist und wobei die Werkzeugelemente miteinander zusammenwirkend angeordnet und ausgebildet sind und eine medizinische Werkzeugeinrichtung bilden, wobei das medizinische Instrument ein vorspannendes Element umfasst, welches mit den zwei Branchen zusammenwirkend angeordnet oder ausgebildet ist.

Medizinische Instrumente sind beispielsweise in Form chirurgischer Instrumente bekannt, an deren proximalen Enden angeordnete Blattfedern, die miteinander gekoppelt sind, das Instrument in einer geöffneten Stellung halten, also in einer Stellung, in der die Werkzeugelemente voneinander weg bewegt sind. In einer kraftneutralen Ruhestellung haben derartige Instrumente insbesondere den Nachteil, dass sie insbesondere in öffnender Richtung keine Spannung aufweisen. Ein solches Instrument liegt dann praktisch "labil" auf einem Tisch. Dies führt dazu, dass ein Anwender beim Aufgreifen des Instruments gefühlt ins Leere greift. Dieser Effekt lässt erst dann nach, wenn ein sogenanntes Blattfedersteckspiel überwunden ist. Dieses Blattfedersteckspiel ergibt sich dadurch, dass die an den freien proximalen Enden der Branchen angeordneten Blattfedern mit ihren freien, von den Branchen weg weisenden Enden ineinandergesteckt sind. Somit halten die zusammenwirkenden Blattfedern derartige chirurgische Instrumente stets in der Offenstellung. Um solche Instrumente in einer geschlossenen Stellung zu halten, werden zusätzlich mechanische Sperren benötigt. Diese müssen dann wiederum mechanisch entsperrt werden, erfordern also eine manuellen Eingriff eines Anwenders.

Es ist daher eine Aufgabe der vorliegenden Erfindung, ein medizinisches Instrument der eingangs beschriebenen Art so zu verbessern, dass es eine anwenderfreundlichere Handhabung ermöglicht.

Diese Aufgabe wird bei einem medizinischen Instrument der eingangs beschriebenen Art erfindungsgemäß dadurch gelöst, dass das vorspannende Element von einer Zustellung, in welcher es eine Zugkraft auf die zwei Branchen ausübt, in eine Druckstellung, in welcher es eine Druckkraft auf die zwei Branchen ausübt, bringbar ist oder umgekehrt durch Änderung seiner Position und/oder seiner Orientierung und/oder seiner Form relativ zu den zwei Branchen.

Die erfindungsgemäß vorgeschlagene Weiterbildung eines medizinischen Instruments ermöglicht es insbesondere, das vorspannende Element wahlweise zu nutzen, um die Werkzeugelemente gegeneinander zu halten, insbesondere unter Vorspannung, oder in einer Stellung voneinander weg. Ein solches Umschalten zwischen der Zugstellung und der Druckstellung erfolgt beispielsweise durch Änderung einer Position des vorspannenden Elements am medizinischen Instrument. Ein solches Umschalten kann aber auch insbesondere durch Änderung einer Orientierung des vorspannenden Elements relativ zu den zwei Branchen erreicht werden beziehungsweise durch Änderung seiner Form relativ zu den zwei Branchen. So lässt sich das Instrument beispielsweise von einer unter Vorspannung gehaltenen geschlossenen Stellung in eine geöffnete Stellung überführen durch Bewegen des vorspannenden Elements relativ zu den zwei Branchen in distaler Richtung. Oder alternativ kann das vorspannende Element relativ zu den zwei Branchen anders ausgerichtet werden, beispielsweise um eine Längsachse des vorspannenden Elements gedreht werden, um die Branchen voneinander weg oder aufeinander zu zu bewegen, je nachdem, ob von der Zugstellung in die Druckstellung umgeschaltet werden soll oder umgekehrt. Durch die vorgeschlagene Weiterbildung ist es insbesondere möglich, auf eine Sperreinrichtung, herkömmlich auch als sogenannte Sperre oder Instrumentensperre bezeichnet, zu verzichten. Insbesondere in der Zugstellung hält das vorspannende Element das Instrument beispielsweise in einer geschlossenen Stellung, in welcher die Klemmelemente aneinander anliegen, insbesondere auch unter einer Vorspannung, die das vorspannende Element ausübt. Beispielsweise kann die Sperrwirkung des vorspannenden Elements aufgehoben werden, indem dieses relativ zu den Branchen anders positioniert oder anders orientiert wird oder indem seine Form geändert wird.

Vorzugsweise ist das vorspannende Element an der ersten Branche und der zweiten Branche gehalten. Auf diese Weise kann das Instrument durch das vorspannende Element insbesondere definiert in einer geöffneten oder in einer geschlossenen Stellung gehalten werden, je nachdem wie dieses relativ zu den Branchen positioniert beziehungsweise orientiert ist beziehungsweise welche Form es aufweist. Das vorspannende Element kann insbesondere lösbar von den Branchen ausgebildet sein, um das Instrument auf einfache Weise zu zerlegen und reinigen zu können.

Günstig ist es, wenn die zwei Branchen in einem Schlussbereich um eine Schwenkachse verschwenkbar aneinander gelagert sind und wenn das vorspannende Element zwischen dem Schlussbereich und den proximalen Enden mit den zwei Branchen zusammenwirkt. Auf diese Weise lassen sich insbesondere medizinische Instrumente herkömmlicher Gestalt, beispielsweise Scheren, Tuchklemmen oder Nadelhalter, durch entsprechende Anordnung und Ausbildung eines vorspannenden Elements in der vorgeschlagenen Weise weiterbilden und gegebenenfalls auch nachrüsten. Die Positionierung des vorspannenden Elements in der vorgeschlagenen Weise hat insbesondere den Vorteil, dass es nicht in unerwünschter Weise mit den Werkzeugelementen kollidieren kann. Insbesondere kann es in der vorgeschlagenen Weise im Bereich von an den Branchen ausgebildeten Griffbereichen angeordnet oder ausgebildet werden. Dies hat insbesondere auch den Vorteil, dass ein Anwender eine Position und/oder einer Orientierung und/oder eine Form des vorspannenden Elements - je nach Ausgestaltung - auch mit der Hand, mit der er das Instrument hält, ändern kann.

Vorteilhaft ist es, wenn vorspannende Element an den Branchen positionsveränderbar, insbesondere verschiebbar, angeordnet ist. Insbesondere kann es sowohl in Richtung auf die distalen Enden hin als auch in Richtung auf die proximalen Enden der zwei Branchen hin positionsveränderbar angeordnet sein. Beispielsweise kann eine Bewegung des vorspannenden Elements in distaler Richtung zu einem Öffnen des Instruments führen, eine Bewegung des vorspannenden Elements in proximaler Richtung zu einem Schließen des Instruments, also zu einer Bewegung der Werkzeugelemente aufeinander zu.

Vorzugsweise ist das vorspannende Element an den zwei Branchen in Richtung auf die Schwenkachse hin und von der Schwenkachse weg positionsveränderbar. So kann es beispielsweise im Bereich eines Griffs des medizinischen Instruments in unterschiedlichen Positionen und/oder Orientierungen und/oder mit einer geänderten Form angeordnet oder positioniert werden, um wahlweise Druck- oder Zugkräfte - oder auch keine Kraft - auf die zwei Branchen auszuüben.

Günstig ist es, wenn das vorspannende Element an den zwei Branchen stufenlos positionsveränderbar oder in diskreten Positionen mit den zwei Branchen zusammenwirkend anordenbar ist. Eine stufenlose Positionsveränderbarkeit hat insbesondere den Vorteil, dass beliebige Öffnungsstellungen und auch beliebige geschlossene Stellungen mit stufenlos einstellbarer Klemmkraft vorgebbar sind. Diskrete Positionen haben insbesondere den Vorteil, dass beispielsweise Schließ- oder Öffnungskräfte in definierter Weise vorgebbar sind.

Gemäß einer weiteren bevorzugten Ausführungsform der Erfindung kann vorgesehen sein, dass das vorspannende Element eine Grundstellung definiert, in welcher die zwei Branchen keine Kraft auf das vorspannende Element ausüben und in welcher das vorspannende Element keine Kraft auf die zwei Branchen ausübt. Die Grundstellung nimmt das vorspannende Element insbesondere nach auch seiner Herstellung ein, wenn es also insbesondere nicht an den Branchen gehalten oder mit diesen in einer anderen Art und Weise gekoppelt ist. In der Grundstellung wirkt insbesondere die Schwerkraft nicht formändernd auf das vorspannende Element.

Günstigerweise weist das vorspannende Element in der Grundstellung eine Grundstellungsform auf. Diese Form kann insbesondere bezüglich einer Längsachse des vorspannenden Elements rotationssymmetrisch sein oder von einer Rotationssymmetrie abweichen. Beispielsweise zum Überführen des vorspannenden Elements von der Zugstellung in die Druckstellung und umgekehrt relativ zu den zwei Branchen ist es vorteilhaft, wenn die Grundstellungsform des vorspannenden Elements rotationsunsymmetrisch ist. Rotationsunsymmetrisch ist beispielsweise eine ovale oder elliptische Form. Ein solches vorspannendes Element weist insbesondere unterschiedliche Ausdehnungen in unterschiedlichen Raumrichtungen aus, insbesondere unterschiedliche Ausdehnungen in zwei voneinander linear unabhängigen Richtungen, wenn das vorspannende Element derart ausgebildet ist, dass es eine Ebene definiert.

Vorzugsweise wirken in der Grundstellung keine äußeren Verformungskräfte auf das vorspannende Element ein. Also weder die Schwerkraft noch insbesondere Kräfte, die von den Branchen auf das vorspannende Element ausübbar wären, führen zu einer Änderung der Grundstellungsform des vorspannenden Elements.

Ferner ist es vorteilhaft, wenn das von mindestens einer der zwei Branchen getrennte vorspannende Element die Grundstellung definiert. Das von den zwei Branchen getrennte vorspannende Element lässt sich so insbesondere leicht reinigen. Zudem kann so auch überprüft werden, ob es beschädigt ist, also insbesondere dauerhaft in seiner Form geändert. Wenn es von den zwei Branchen getrennt ist, können diese keine Kräfte auf das vorspannende Element ausüben. Die auf das vorspannende Element wirkende Schwerkraft bleibt hier insbesondere unberücksichtigt.

Günstig ist es, wenn in einer Neutralstellung des Instruments die zwei Werkzeugelemente aneinander anliegen, ohne eine Kraft aufeinander auszuüben. So lässt sich eine definierte Stellung des Instruments vorgeben, nämlich eine geschlossene Stellung. Ein Anwender kann es ergreifen, wobei es in der Neutralstellung spielfrei ist. Die beiden Branchen weisen also in der Neutralstellung kein Spiel auf, da die Werkzeugelemente aneinander anliegen.

Vorzugsweise nimmt das vorspannende Element in der Neutralstellung des Instruments die Grundstellung ein. In dieser übt es dann keine Kraft auf die zwei Branchen aus. Das Instrument befindet sich in der Neutralstellung, wenn das vorspannende Element die Grundstellung einnimmt, in einem kraftneutralen Zustand.

Vorteilhaft ist es, wenn die zusammenwirkenden Werkzeugelemente ausgehend von der Neutralstellung entgegen der Wirkung des vorspannenden Elements voneinander weg bewegbar sind. Beispielsweise können die Branchen hierzu voneinander weg bewegt werden, sodass das vorspannende Element eine Zugkraft auf die zwei Branchen ausübt, um sie wieder in die Neutralstellung zurückzubewegen.

Ferner kann es günstig sein, wenn das Instrument von der Neutralstellung in eine Aufnahmestellung, in welcher die zusammenwirkenden Werkzeugelement durch das vorspannende Element voneinander weg bewegt sind, insbesondere voneinander weg verschwenkt, bringbar ist durch Änderung seiner Position und/oder seiner Orientierung und/oder seiner Form relativ zu den zwei Branchen. Mit anderen Worten kann das Instrument von der Neutralstellung in die Aufnahmestellung überführt werden. Es kann also geöffnet werden durch Bewegen der Werkzeugelemente voneinander weg. Dies kann beispielsweise erreicht werden, indem das vorspannende Element relativ zu den zwei Branchen bewegt oder anders angeordnet wird und/oder seine Orientierung geändert wird relativ zu den zwei Branchen und/oder seine Form relativ zu den zwei Branchen geändert wird. Beim Übergang von der Neutralstellung in die Aufnahmestellung kann das vorspannende Instrument insbesondere von der Grundstellung in die Zugstellung überführt werden. Es kann jedoch auch die Grundstellung beibehalten.

Vorzugsweise behält das vorspannende Element beim Übergang des Instruments von der Neutralstellung in die Aufnahmestellung die Grundstellung bei. Dies hat dann insbesondere den Vorteil, dass das vorspannende Element in dieser Aufnahmestellung keine Kräfte auf die zwei Branchen ausübt. Allerdings können die Werkzeugelemente durch Ausüben von Kräften auf die zwei Branchen und damit indirekt auf das vorspannende Element entgegen der Wirkung des vorspannenden Elements aufeinander zu oder voneinander weg bewegt werden.

Günstig ist es, wenn die zusammenwirkenden Werkzeugelemente in der Aufnahmestellung entgegen der Wirkung des vorspannenden Elements aufeinander zu bewegbar sind. So kann durch das vorspannende Element eine erforderliche Schließkraft für das medizinische Instrument vorgegeben werden. Durch eine entsprechende Wahl des vorspannenden Elements kann so das medizinische Instrument für einen Anwender insbesondere individualisiert werden, um eine optimale Anpassung an dessen Feinmotorik zu ermöglichen. Bei dem beschriebenen Schließen des Instruments wird das vorspannende Element von der Grundstellung in die Druckstellung überführt.

Vorteilhaft ist es, wenn das Instrument von der Neutralstellung in eine Klemmstellung, in welcher die zusammenwirkenden Werkzeugelement durch das vorspannende Element unter Vorspannung gegeneinander gehalten sind, bringbar ist durch Überführen des vorspannenden Elements von der Neutralstellung in die Zugstellung durch Änderung seiner Position und/oder seiner Orientierung und/oder seiner Form relativ zu den zwei Branchen. Durch Änderung von Position und/oder Orientierung und/oder Form des vorspannenden Elements kann so auch in gezielter Weise eine vorspannende Kraft, mit welcher die Werkzeugelemente gegeneinander gedrückt werden, vorgegeben werden. Insbesondere ist in dieser Stellung, also in der Klemmstellung, das vorspannende Element funktional vergleichbar mit einer aktivierten Sperreinrichtung. Daher ist bei einem solchen Instrument eine Sperreinrichtung insbesondere überflüssig, da die Funktion der Sperreinrichtung wie beschrieben durch das vorspannende Element ausgeübt werden kann. Beispielsweise lässt sich das Instrument von der Neutralstellung in eine Klemmstellung überführen, und zwar dadurch, dass das vorspannende Element seine Position ändert, und zwar in proximaler Richtung. Insbesondere bei verschwenkbar aneinandergelagerten Branchen kann so bei stufenlosem Verschieben des vorspannenden Elements in proximaler Richtung an den zwei Branchen eine Klemmkraft kontinuierlich erhöht werden. So lassen sich beispielsweise Gewebe oder auch Tücher in definierter Weise zwischen den zwei Werkzeugelementen klemmen.

Überdies kann es günstig sein, wenn das das vorspannende Element eine Längsachse definiert und wenn es in der Grundstellung in zwei voneinander linear unabhängigen Richtungen, welche quer, insbesondere senkrecht, zur Längsachse orientiert sind, eine erste Ausdehnung und eine zweite Ausdehnung aufweist, wenn die erste Ausdehnung kleiner ist als die zweite Ausdehnung und wenn in der Neutralstellung oder in der Klemmstellung das vorspannende Element mit den zwei Branchen in Richtung der ersten Ausdehnung an den zwei Branchen gehalten ist. Eine solche Ausgestaltung ermöglicht es insbesondere, durch Ändern der Orientierung des vorspannenden Elements das Instrument von der Neutralstellung in die Aufnahmestellung zu überführen oder von der Klemmstellung in die Neutralstellung und dann von dieser in die Aufnahmestellung. Auch eine umgekehrte Änderung der Stellungen ist beispielsweise möglich. Durch Ändern der Orientierung lässt sich aufgrund der unterschiedlichen Ausdehnungen des vorspannenden Elements in unterschiedlichen Richtungen insbesondere ein Abstand der zwei Branchen relativ zueinander ändern. Beispielsweise bei verschwenkbar aneinander gelagerten Brachen können so die Werkzeugelemente voneinander weg oder aber auch aufeinander zu bewegt werden durch Ändern einer Orientierung des vorspannenden Elements relativ zu den zwei Branchen.

Auf einfache Weise lassen sich Stellungen des Instruments ändern, wenn das Instrument durch Änderung der Orientierung des vorspannenden Elements, nämlich insbesondere durch Drehung um die Längsachse, von der Neutralstellung in die Aufnahmestellung und umgekehrt oder von der Neutralstellung in die Klemmstellung und umgekehrt bringbar ist. Mit anderen Worten muss bei einem solchen Instrument lediglich eine Orientierung des vorspannenden Elements geändert werden, beispielsweise durch Drehung um seine Längsachse, um das Instrument zu öffnen oder zu schließen. Je nach gewünschtem Einsatzzweck können so die Klemmelemente aneinander anliegen, insbesondere unter Vorspannung, oder voneinander weg gehalten werden.

Weiterhin ist es vorteilhaft, wenn das Instrument durch Änderung der Form des vorspannenden Elements von der Neutralstellung in die Aufnahmestellung und umgekehrt oder von der Neutralstellung in die Klemmstellung und umgekehrt bringbar ist. Beispielsweise kann die Änderung der Form durch Vergrößern der ersten Ausdehnung und/oder Verringern der zweiten Ausdehnung erreicht werden. Das Überführen des Instruments von der Neutralstellung in die Klemmstellung kann insbesondere erreicht werden durch Verringern der ersten Ausdehnung und/oder Vergrößern der zweiten Ausdehnung. Eine solche Formänderung kann beispielsweise bei einem ovalen vorspannenden Element erreicht werden, welches unterschiedliche Ausdehnungen in zwei zueinander senkrechten Richtungen aufweist, indem es in der schmaleren Richtung verschmälert wird, wodurch es dann in der anderen Richtung etwas vergrößert wird oder eben umgekehrt. So ist es möglich, durch Ändern der Form des vorspannenden Elements die Branchen, mit denen es zusammenwirkt, durch Formänderung entweder voneinander weg zu bewegen oder aufeinander zu oder, wenn sie schon in einer maximal angenäherten Stellung sind, unter Vorspannung gegeneinander zu halten.

Vorzugsweise umfasst das Instrument eine Formänderungseinrichtung umfasst zum Ändern der Form des vorspannenden Elements. Dies ermöglicht es auf einfache Weise, die Form des vorspannenden Elements zu ändern. Hierzu muss lediglich die Formänderungseinrichtung betätigt werden.

Günstig ist es, wenn die die Formänderungseinrichtung mit dem vorspannenden Element zusammenwirkend angeordnet oder ausgebildet ist zum Vergrößern der ersten Ausdehnung und/oder Verringern der zweiten Ausdehnung.

Beispielsweise ist es möglich, mit der Formänderungseinrichtung die eine Ausdehnung zu vergrößern und die andere zu verkleinern und umgekehrt.

Vorteilhaft ist es, wenn die Formänderungseinrichtung in Form einer in Richtung der ersten Ausdehnung oder der zweiten Ausdehnung wirkenden Sperre ausgebildet ist. So kann die Form des vorspannenden Elements verändert werden, durch Aktivieren der Sperre aktiviert, beispielsweise durch Verringern der ersten Ausdehnung oder der zweiten Ausdehnung. Insbesondere kann die Sperre zwei zusammenwirkende, aufeinander zu weisende Sperrhaken umfassen, die in der Neutralstellung und/oder der Klemmstellung außer Eingriff stehen und in der Aufnahmestellung in Eingriff stehen. Beispielsweise kann so durch Aktivieren der Sperre, nämlich in Eingriff Bringen der zusammenwirkenden Sperrhaken, das Instrument von der Neutralstellung in die Aufnahmestellung überführt werden.

Günstig ist es, wenn die Formänderungseinrichtung ein in seiner Länge veränderbares Band umfasst, welches das vorspannende Element in der Grundstellung in Richtung der zweiten Ausdehnung umgibt, und wenn durch Verkürzen des Bands das Instrument von der Neutralstellung und/oder der Klemmstellung in die Aufnahmestellung bringbar ist. Beispielsweise kann das Band derart angeordnet sein, dass es durch Ausübung einer Zugkraft auf das Band seine Länge verändert und dadurch die Form des vorspannenden Elements ändert. Ist die zweite Ausdehnung größer als die erste Ausdehnung, kann so durch Verkürzen des Bands die zweite Ausdehnung verringert und die erste Ausdehnung vergrößert werden.

Auf besonders einfache Weise ausbilden lässt sich das medizinische Instrument, wenn das Band in Form eines Kabelbinders ausgebildet ist. Eine eines solchen Kabelbinders kann insbesondere unidirektional auf einfache Weise geändert werden, indem an einem freien Ende desselben gezogen wird. So kann beispielsweise das Instrument durch einen Anwender von der Klemmstellung in die Neutralstellung und von der Neutralstellung in die Aufnahmestellung überführt werden durch einfaches Ziehen am freien Ende des Kabelbinders.

Günstigerweise sind die Werkzeugelemente in Form von Klemmelementen ausgebildet. So kann beispielsweise ein Instrument ausgebildet werden, dessen Werkzeugeinrichtung als Klemmeinrichtung ausgebildet ist.

Vorteilhaft ist es, wenn jedes Klemmelement einer Klemmfläche aufweist und wenn die Klemmflächen in der Neutralstellung aneinander anliegen. Insbesondere können die Klemmflächen so geformt werden, dass sie ein zu klemmendes Gut, beispielsweise Körpergewebe oder ein Tuch, optimal zwischen sich halten können.

Vorzugsweise sind die Klemmflächen makroskopisch strukturiert. Auf diese Weise kann insbesondere ein Abrutschen von Körpergewebe oder von Tüchern von den Klemmflächen verhindert werden.

Gemäß einer weiteren bevorzugten Form der Erfindung kann vorgesehen sein, dass das vorspannende Element in Form eines federelastischen Elements und/oder in Form eines gummielastischen Elements ausgebildet ist. Derartige vorspannende Elemente können insbesondere sowohl auf Zug als auch auf Druck beansprucht werden. Zudem können sie in unterschiedlichsten Formen bereitgestellt werden, insbesondere auch derart, dass sie ihre Form ändern können, beispielsweise mittels einer Formänderungseinrichtung.

Auf einfache Weise lässt sich das Instrument ausbilden, wenn das federelastische Element in Form einer Schraubenfeder oder einer in sich geschlossenen Ringfeder ausgebildet ist. Die Schraubenfeder ist vorzugsweise derart ausgebildet, dass sie sowohl zusammengedrückt als auch auseinandergezogen werden kann. Mithin ist es vorteilhaft, wenn die Windungen der Schraubenfeder in der Grundstellung nicht aneinander anliegen, sodass die Schraubenfeder sowohl komprimiert als auch auseinandergezogen werden kann. Eine in sich geschlossene Ringfeder kann insbesondere aus einem flachen Blechstreifen oder auch aus einem im Querschnitt kreisförmigen, ovalen oder rechteckigen Draht ausgebildet sein.

Besonders einfach ausbilden lässt sich das vorspannende Element, wenn die Ringfeder in Form eines schmalen, in sich geschlossenen Federbandes ausgebildet ist und wenn eine Breite des Federbandes größer ist als eine Dicke desselben. Ein solches Federband lässt sich insbesondere in unterschiedlichen Richtungen leicht zusammendrücken, wodurch insbesondere seine Form geändert wird. Zudem kann es durch ein Zusammendrücken beispielsweise von der Grundstellung in die Druckstellung überführt werden. Ein solches Federelement kann beispielsweise an die zwei Branchen angekoppelt werden und diese dann aber auch unter Zug halten, wofür das Federelement zum Koppeln mit den beiden Branchen bei geschlossenem Instrument beispielsweise nur von der Grundstellung in die Zugstellung überführt werden muss.

Vorteilhaft ist es, wenn das das vorspannende Element eine Längsachse definiert und dass es in der Grundstellung in einer Ebene quer, insbesondere senkrecht, zur Längsachse oval, insbesondere kreisförmig, geformt ist. Eine ovale Form ermöglicht insbesondere durch Änderung der Orientierung des vorspannenden Elements eine Überführung des Instruments von der Neutralstellung in die Klemmstellung oder von der Neutralstellung in die Aufnahmestellung.

Ferner kann es günstig sein, wenn das gummielastische Element in Form eines Gummikörpers ausgebildet ist. Insbesondere kann dieser stabförmig ausgebildet sein. Beispielsweise sind voneinander weg weisende Enden des Gummikörpers jeweils an einer der zwei Branchen angeordnet. Auch ein solcher Gummikörper kann wahlweise auf Zug oder Druck belastet werden, mithin also von einer Grundstellung in eine Zugstellung beziehungsweise in eine Druckstellung überführt werden.

Vorzugsweise ist das vorspannende Element aus einem Metall, insbesondere aus einem Federstahl, oder aus einem Kunststoff ausgebildet. Aus den genannten Materialien lassen sich insbesondere vorspannende Elemente unterschiedlichster Formen realisieren. Zudem eignen sich diese Materialien auch zur Herstellung medizinischer Instrumente und heißdampfsterilisierbar.

Gemäß einer weiteren bevorzugten Ausführungsform der Erfindung kann vorgesehen sein, dass die medizinische Werkzeugeinrichtung in Form einer Schneideinrichtung oder in Form einer Klemmeinrichtung ausgebildet ist. So können insbesondere Scheren oder Klemmen ausgebildet werden.

Günstig ist es, wenn das medizinische Instrument in Form eines Ringinstruments ausgebildet ist und an den proximalen Enden der zwei Branchen ein Ring angeordnet oder ausgebildet ist. Derartige Instrumente lassen sich von einem Anwender optimal handhaben. Beispielsweise kann beim Betätigen des Instruments in jeden Ring ein Finger des Anwenders eingreifen.

Günstig ist es, wenn das medizinische Instrument in Form eines Nadelhalters, einer Schere oder einer medizinischen Klemme, insbesondere einer Tuchklemme, ausgebildet ist. Derartige Instrumente können durch die besondere Ausgestaltung mit dem vorspannenden Element einfach und sicher gehandhabt werden.

Gemäß einer weiteren bevorzugten Ausführungsform der Erfindung kann vorgesehen sein, dass das medizinische Instrument modular ausgebildet ist und mindestens zwei sich in Form und/oder Größe und/oder in ihrer vorspannenden Charakteristik unterscheidende vorspannende Elemente umfasst, welche wahlweise mit den zwei Branchen koppelbar sind. Mithin kann also ein medizinisches Instrumentensystem bereitgestellt werden mit unterschiedlichen Instrumenten und mindestens zwei unterschiedlichen vorspannenden Elementen. So lassen sich die Instrumente durch geeignete Auswahl eines vorspannenden Elements aus den verfügbaren vorspannenden Elementen für einen Anwender individualisieren. Durch Bereitstellen unterschiedlicher vorspannender Elemente, die sich in ihrer vorspannenden Charakteristik unterscheiden, beispielsweise einem E-Modul, können so Klemm- oder Schließkräfte des Instruments in gewünschter Weise vorgegeben werden. Durch unterschiedliche Größen können beispielsweise Öffnungsweiten des Instruments in der Öffnungsstellung auf einfache Weise vorgegeben werden.

Die vorstehende Beschreibung umfasst somit insbesondere die nachfolgend in Form durchnummerierter Sätze definierten Ausführungsformen medizinischer Instrumente:
1. Medizinisches Instrument (10) mit zwei relativ zueinander bewegbaren Branchen (12, 14), wobei jede Branche (12, 14) ein distales Ende (16, 18) mit einem Werkzeugelement (32, 34) und ein proximales Ende (20, 22) aufweist und wobei die Werkzeugelemente (32, 34) miteinander zusammenwirkend angeordnet und ausgebildet sind und eine medizinische Werkzeugeinrichtung (36) bilden, wobei das medizinische Instrument (10) ein vorspannendes Element (38) umfasst, welches mit den zwei Branchen (12, 14) zusammenwirkend angeordnet oder ausgebildet ist, dadurch gekennzeichnet, dass das vorspannende Element (38) von einer Zugstellung, in welcher es eine Zugkraft auf die zwei Branchen (12, 14) ausübt, in eine Druckstellung, in welcher es eine Druckkraft auf die zwei Branchen (12, 14) ausübt, bringbar ist oder umgekehrt durch Änderung seiner Position und/oder seiner Orientierung und/oder seiner Form relativ zu den zwei Branchen (12, 14).
2. Medizinisches Instrument nach Satz 1, dadurch gekennzeichnet, dass das vorspannende Element (38) an der ersten Branche (12) und der zweiten Branche (14) gehalten ist.
3. Medizinisches Instrument nach einem der voranstehenden Sätze, dadurch gekennzeichnet, dass die zwei Branchen (12, 14) in einem Schlussbereich (26) um eine Schwenkachse (24) verschwenkbar aneinander gelagert sind und dass das vorspannende Element (38) zwischen dem Schlussbereich (26) und den proximalen Enden (20, 22) mit den zwei Branchen (12, 14) zusammenwirkt.
4. Medizinisches Instrument nach einem der voranstehenden Sätze, dadurch gekennzeichnet, dass das vorspannende Element (38) an den Branchen (12, 14) positionsveränderbar, insbesondere verschiebbar, angeordnet ist, insbesondere sowohl in Richtung auf die distalen Enden (16, 18) hin als auch in Richtung auf die proximalen Enden (20, 22) der zwei Branchen hin (12, 14).
5. Medizinisches Instrument nach Satz 4, dadurch gekennzeichnet, dass das vorspannende Element (38) an den zwei Branchen (12, 14) in Richtung auf die Schwenkachse (24) hin und von der Schwenkachse (24) weg positionsveränderbar ist.
6. Medizinisches Instrument nach Satz 4 oder 5, dadurch gekennzeichnet, dass das vorspannende Element (38) an den zwei Branchen (12, 14) stufenlos positionsveränderbar oder in diskreten Positionen mit den zwei Branchen (12, 14) zusammenwirkend anordenbar ist.
7. Medizinisches Instrument nach einem der voranstehenden Sätze, dadurch gekennzeichnet, dass das vorspannende Element (38) eine Grundstellung definiert, in welcher die zwei Branchen (12, 14) keine Kraft auf das vorspannende Element (38) ausüben und in welcher das vorspannende Element (38) keine Kraft auf die zwei Branchen (12, 14) ausübt.
8. Medizinisches Instrument nach Satz 7, dadurch gekennzeichnet, dass das vorspannende Element (38) in der Grundstellung eine Grundstellungsform aufweist.
9. Medizinisches Instrument nach Satz 7 oder 8, dadurch gekennzeichnet, dass in der Grundstellung keine äußeren Verformungskräfte auf das vorspannende Element (38) einwirken.
10. Medizinisches Instrument nach einem der Sätze 7 bis 9, dadurch gekennzeichnet, dass das von mindestens einer der zwei Branchen (12, 14) getrennte vorspannende Element (38) die Grundstellung definiert.
11. Medizinisches Instrument nach einem der Sätze 7 bis 10, dadurch gekennzeichnet, dass in einer Neutralstellung des Instruments (10) die zwei Werkzeugelemente (32, 34) aneinander anliegen, ohne eine Kraft aufeinander auszuüben.
12. Medizinisches Instrument nach Satz 11, dadurch gekennzeichnet, dass das vorspannende Element (38) in der Neutralstellung des Instruments (10) die Grundstellung einnimmt.
13. Medizinisches Instrument nach Satz 11 oder 12, dadurch gekennzeichnet, dass die zusammenwirkenden Werkzeugelemente (32, 34) ausgehend von der Neutralstellung entgegen der Wirkung des vorspannenden Elements (38) voneinander weg bewegbar sind.
14. Medizinisches Instrument nach einem der Sätze 11 bis 13, dadurch gekennzeichnet, dass das Instrument von der Neutralstellung in eine Aufnahmestellung, in welcher die zusammenwirkenden Werkzeugelement (32, 34) durch das vorspannende Element (38) voneinander weg bewegt sind, insbesondere voneinander weg verschwenkt, bringbar ist durch Änderung seiner Position und/oder seiner Orientierung und/oder seiner Form relativ zu den zwei Branchen.
15. Medizinisches Instrument nach Satz 14, dadurch gekennzeichnet, dass das vorspannende Element (38) beim Übergang des Instruments (10) von der Neutralstellung in die Aufnahmestellung die Grundstellung beibehält.
16. Medizinisches Instrument nach Satz 14 oder 15, dadurch gekennzeichnet, dass die zusammenwirkenden Werkzeugelemente (32, 34) in der Aufnahmestellung entgegen der Wirkung des vorspannenden Elements (38) aufeinander zu bewegbar sind.
17. Medizinisches Instrument nach einem der Sätze 11 bis 16, dadurch gekennzeichnet, dass das Instrument von der Neutralstellung in eine Klemmstellung, in welcher die zusammenwirkenden Werkzeugelemente (32, 34) durch das vorspannende Element (38) unter Vorspannung gegeneinander gehalten sind, bringbar ist durch Überführen des vorspannenden Elements (38) von der Neutralstellung in die Zugstellung durch Änderung seiner Position und/oder seiner Orientierung und/oder seiner Form relativ zu den zwei Branchen (12, 14).
18. Medizinisches Instrument nach einem der Sätze 11 bis 17, dadurch gekennzeichnet, dass das vorspannende Element (38) eine Längsachse (70) definiert und dass es in der Grundstellung in zwei voneinander linear unabhängigen Richtungen, welche quer, insbesondere senkrecht, zur Längsachse orientiert sind, eine erste Ausdehnung (72) und eine zweite Ausdehnung (74) aufweist, dass die erste Ausdehnung (72) kleiner ist als die zweite Ausdehnung (74) und dass in der Neutralstellung oder in der Klemmstellung das vorspannende Element (38) mit den zwei Branchen (12, 14) in Richtung der ersten Ausdehnung (72) an den zwei Branchen (12, 14) gehalten ist.
19. Medizinisches Instrument nach Satz 18, dadurch gekennzeichnet, dass das Instrument (10) durch Änderung der Orientierung des vorspannenden Elements (38), nämlich insbesondere durch Drehung um die Längsachse (70), von der Neutralstellung in die Aufnahmestellung und umgekehrt oder von der Neutralstellung in der Klemmstellung und umgekehrt bringbar ist.
20. Medizinisches Instrument nach Satz 18 oder 19, dadurch gekennzeichnet, dass das Instrument (10) durch Änderung der Form des vorspannenden Elements (38), nämlich insbesondere durch Vergrößern der ersten Ausdehnung (72) und/oder Verringern der zweiten Ausdehnung (74), von der Neutralstellung in die Aufnahmestellung und umgekehrt oder von der Neutralstellung in die Klemmstellung, nämlich insbesondere durch Verringern der ersten Ausdehnung (72) und/oder Vergrößern der zweiten Ausdehnung (74), und umgekehrt bringbar ist.
21. Medizinisches Instrument nach einem der voranstehenden Sätze, dadurch gekennzeichnet, dass das Instrument (10) eine Formänderungseinrichtung (82) umfasst zum Ändern der Form des vorspannenden Elements (38).
22. Medizinisches Instrument nach Satz 21, dadurch gekennzeichnet, dass die Formänderungseinrichtung (82) mit dem vorspannenden Element (38) zusammenwirkend angeordnet oder ausgebildet ist zum Vergrößern der ersten Ausdehnung (72) und/oder Verringern der zweiten Ausdehnung (74).
23. Medizinisches Instrument nach Satz 21 oder 22, dadurch gekennzeichnet, dass die Formänderungseinrichtung (82) in Form einer in Richtung der ersten Ausdehnung (72) oder der zweiten Ausdehnung (74) wirkenden Sperre (94) ausgebildet ist, insbesondere umfassend zwei zusammenwirkende, aufeinander zu weisende Sperrhaken (96, 98), die in der Neutralstellung und/oder der Klemmstellung außer Eingriff stehen und in der Aufnahmestellung in Eingriff stehen.
24. Medizinisches Instrument nach einem der Sätze 21 bis 23, dadurch gekennzeichnet, dass die Formänderungseinrichtung (82) ein in seiner Länge veränderbares Band (84) umfasst, welches das vorspannende Element (38) in der Grundstellung in Richtung der zweiten Ausdehnung (74) umgibt, und dass durch Verkürzen des Bands (84) das Instrument (10) von der Neutralstellung und/oder der Klemmstellung in die Aufnahmestellung bringbar ist.
25. Medizinisches Instrument nach Satz 24, dadurch gekennzeichnet, dass das Band (84) in Form eines Kabelbinders (90) ausgebildet ist.
26. Medizinisches Instrument nach einem der voranstehenden Sätze, dadurch gekennzeichnet, dass die Werkzeugelemente (32, 34) in Form von Klemmelementen (54, 56) ausgebildet sind.
27. Medizinisches Instrument nach Satz 26, dadurch gekennzeichnet, dass jedes Klemmelement (54,5 6) eine Klemmfläche (58, 60) aufweist und dass die Klemmflächen (58, 60) in der Neutralstellung aneinander anliegen.
28. Medizinisches Instrument nach Satz 27, dadurch gekennzeichnet, dass die Klemmflächen (58, 60) makroskopisch strukturiert sind.
29. Medizinisches Instrument nach einem der voranstehenden Sätze, dadurch gekennzeichnet, dass das vorspannende Element (38) in Form eines federelastischen Elements (44) und/oder in Form eines gummielastischen Elements (100) ausgebildet ist.
30. Medizinisches Instrument nach Satz 29, dadurch gekennzeichnet, dass das federelastische Element (44) in Form einer Schraubenfeder oder einer in sich geschlossenen Ringfeder (46) ausgebildet ist.
31. Medizinisches Instrument nach Satz 30, dadurch gekennzeichnet, dass die Ringfeder (46) in Form eines schmalen, in sich geschlossenen Federbandes (48) ausgebildet ist und dass eine Breite (50) des Federbandes (48) größer ist als eine Dicke (52) desselben.
32. Medizinisches Instrument nach einem der Sätze 7 bis 31, dadurch gekennzeichnet, dass das vorspannende Element (38) eine Längsachse (70) definiert und dass es in der Grundstellung in einer Ebene quer, insbesondere senkrecht, zur Längsachse (70) oval, insbesondere kreisförmig, geformt ist.
33. Medizinisches Instrument nach einem der Sätze 29 bis 32, dadurch gekennzeichnet, dass das gummielastische Element (100) in Form eines, insbesondere stabförmigen, Gummikörpers (102) ausgebildet ist, wobei insbesondere voneinander weg weisende Enden (104, 106) des Gummikörpers (102) jeweils an einer der zwei Branchen (12, 14) angeordnet sind.
34. Medizinisches Instrument nach einem der voranstehenden Sätze, dadurch gekennzeichnet, dass das vorspannende Element (38) aus einem Metall, insbesondere aus einem Federstahl, oder aus einem Kunststoff ausgebildet ist.
35. Medizinisches Instrument nach einem der voranstehenden Sätze, dadurch gekennzeichnet, dass die medizinische Werkzeugeinrichtung (36) in Form einer Schneideinrichtung oder in Form einer Klemmeinrichtung (112) ausgebildet ist.
36. Medizinisches Instrument nach einem der voranstehenden Sätze, dadurch gekennzeichnet, dass das medizinische Instrument (10) in Form eines Ringinstruments (114) ausgebildet ist und an den proximalen Enden (20, 22) der zwei Branchen (12, 14) ein Ring (28, 30) angeordnet oder ausgebildet ist.
37. Medizinisches Instrument nach einem der voranstehenden Sätze, dadurch gekennzeichnet, dass das medizinische Instrument (10) in Form eines Nadelhalters, einer Schere oder einer medizinischen Klemme, insbesondere einer Tuchklemme, ausgebildet ist.
38. Medizinisches Instrument nach einem der voranstehenden Sätze, dadurch gekennzeichnet, dass das medizinische Instrument (10) modular ausgebildet ist und mindestens zwei sich in Form und/oder Größe und/ oder in ihrer vorspannenden Charakteristik unterscheidende vorspannende Elemente (38) umfasst, welche wahlweise mit den zwei Branchen (12, 14) koppelbar sind.

Die nachfolgende Beschreibung bevorzugter Ausführungsformen der Erfindung dient im Zusammenhang mit den Zeichnungen der näheren Erläuterungen. Es zeigen:
- Figur 1:: eine schematische perspektivische Gesamtansicht eines Ausführungsbeispiels eines medizinischen Instruments;
- Figur 2:: eine schematische Draufsicht auf das Ausführungsbeispiel aus Figur 1, wobei das Instrument die Neutralstellung einnimmt und das vorspannende Element die Grundstellung;
- Figur 3:: eine schematische Ansicht der Anordnung aus Figur 2, jedoch mit dem Instrument in der Klemmstellung und dem vorspannenden Element in der Zugstellung;
- Figur 4:: eine schematische Ansicht eines Teils des Instruments aus Figur 2, jedoch in der Aufnahmestellung mit vorspannendem Element in der Grundstellung;
- Figur 5:: eine schematische Draufsicht auf das Instrument aus Figur 4, jedoch mit aufeinander zu bewegten Branchen und dem vorspannenden Element in der Druckstellung;
- Figur 6:: eine vergrößerte Teilansicht des Bereichs A in Figur 3;
- Figur 7:: eine vergrößerte Teilansicht des Bereichs B in Figur 5;
- Figur 8:: eine schematische Ansicht ähnlich Figur 2 eines weiteren Ausführungsbeispiels eines medizinischen Instruments in der Neutralstellung mit dem vorspannenden Element in der Grundstellung;
- Figur 9:: eine Ansicht des Instruments aus Figur 8 in der Klemmstellung mit dem vorspannenden Element in der Zugstellung;
- Figur 10:: eine schematische Draufsicht auf das Instrument aus Figur 8 in der Aufnahmestellung mit dem vorspannenden Element in der Grundstellung, jedoch bei geänderter Orientierung desselben;
- Figur 11:: eine schematische Ansicht der Anordnung aus Figur 10 mit auf die Branchen wirkenden Kräften und dem vorspannenden Element in der Druckstellung;
- Figur 12:: eine vergrößerte Teilansicht des Bereichs C in Figur 9;
- Figur 13:: eine vergrößerte Teilansicht des Bereichs D aus Figur 11;
- Figur 14:: eine schematische Draufsicht auf ein weiteres Ausführungsbeispiel eines medizinischen Instruments mit vorspannendem Element und Formänderungseinrichtung;
- Figur 15:: eine schematische Draufsicht auf ein weiteres Ausführungsbeispiel eines vorspannenden Elements mit Formänderungseinrichtung;
- Figur 16:: eine schematische Draufsicht auf das Instrument aus Figur 14 in der Aufnahmestellung mit in seiner Form geändertem vorspannenden Element;
- Figur 17:: eine schematische Draufsicht auf das Instrument aus Figur 16 mit auf die Branchen wirkenden Kräften und vorspannendem Element in der Druckstellung;
- Figur 18:: eine schematische vergrößerte Teilansicht ähnlich Figur 13 eines weiteren Ausführungsbeispiels eines medizinischen Instruments mit einem vorspannenden Element in Form eines gummielastischen Körpers; und
- Figur 19:: eine Ansicht ähnlich Figur 18 eines weiteren Ausführungsbeispiels eines medizinischen Instruments mit schematischer Darstellung des vorspannenden Elements umfassend ein Druckelement und ein Zugelement.

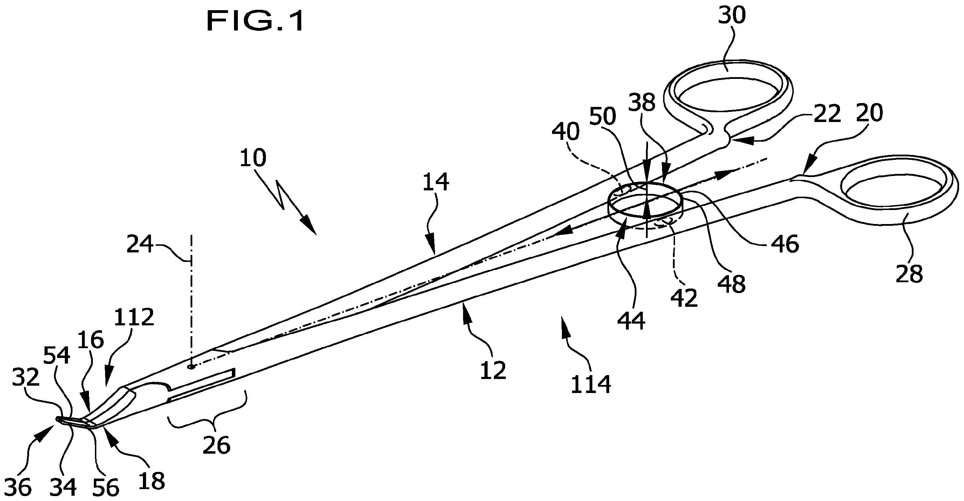

In Figur 1 ist schematisch ein erstes Ausführungsbeispiel eines insgesamt mit dem Bezugszeichen 10 bezeichneten medizinischen Instruments schematisch dargestellt. Es umfasst zwei Branchen 12 und 14, welche jeweils ein distales Ende 16 beziehungsweise 18 und ein proximales Ende 20 beziehungsweise 22 aufweisen.

Die beiden Branchen 12 und 14 sind relativ zueinander beweglich angeordnet.

Bei dem in Figur 1 schematisch dargestellten Ausführungsbeispiel sind die Branchen 12 und 14 in einem Schlussbereich 26 verschwenkbar um eine Schwenkachse 24 aneinander gelagert.

Bei dem dargestellten Ausführungsbeispiel sind an den proximalen Enden 20 und 22 Fingerringe 28 beziehungsweise 30 angeordnet. Die beiden Fingerringe 28 und 30 definieren eine gemeinsame Instrumentenebene, welche senkrecht zur Schwenkachse 24 verläuft.

An den distalen Enden 16 und 18 sind Werkzeugelemente 32 beziehungsweise 34 angeordnet beziehungsweise ausgebildet. Die beiden Werkzeugelemente 32 und 34 wirken zusammen und bilden eine medizinische Werkzeugeinrichtung 36.

Die Branchen 12 und 14 erstrecken sich vom Schlussbereich 26 bei dem dargestellten Ausführungsbeispiel geradlinig in proximaler Richtung.

Das Instrument 10 umfasst ferner ein vorspannendes Element 38. Das vorspannende Element 38 wirkt mit den beiden Branchen 12 und 14 zusammen. Hierfür ist es mit an jeweils einem Haltepunkt 40 beziehungsweise 42 an den Branchen 12 und 14 gehalten.

Das vorspannende Element 38 ist bei dem dargestellten Ausführungsbeispiel in Form eines federelastischen Elements 44 ausgebildet, und zwar in Form einer in sich geschlossenen Ringfeder 46. Die Ringfeder 46 ist in Form eines schmalen, in sich geschlossenen Federbandes 48 ausgebildet. Eine Breite 50 des Federbandes 48 ist größer als eine Dicke 52 desselben.

Das vorspannende Element 38 ist bei dem in den Figuren 1 bis 7 dargestellten Ausführungsbeispiel des Instruments 10 zwischen dem Schlussbereich 26 und den proximalen Enden 20 und 22 mit den zwei Branchen 12 und 14 zusammenwirkend angeordnet.

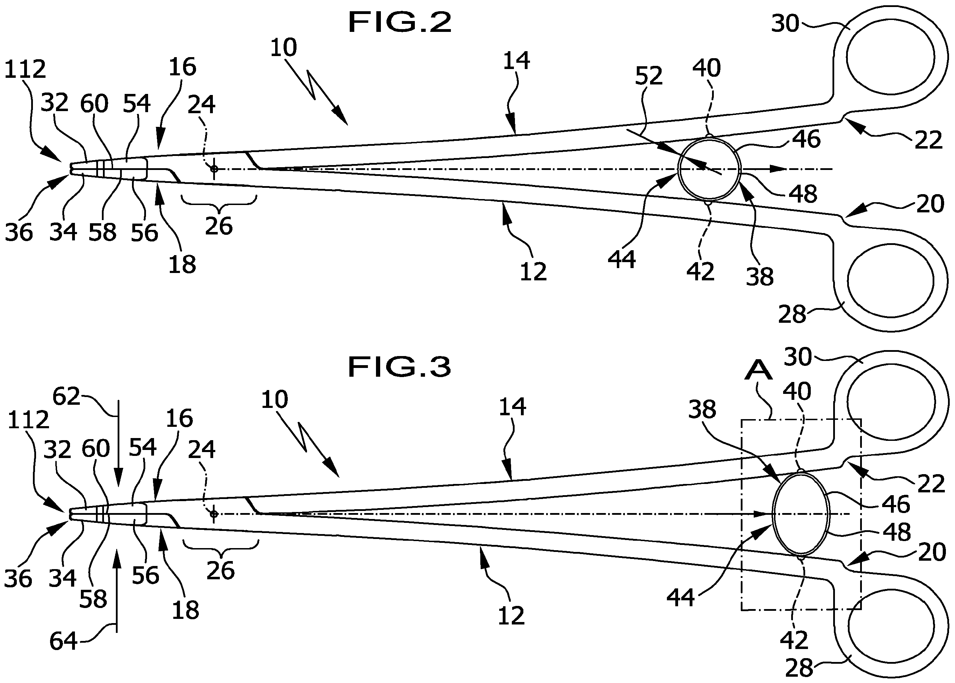

Die Eigenschaften sowie die Funktion des vorspannenden Elements 38 werden nachfolgend in Verbindung mit den Figuren 2 bis 7 näher erläutert.

In Figur 2 ist das Instrument 10 schematisch in einer Neutralstellung dargestellt. Die beiden Werkzeugelemente 32 und 34 sind bei diesem Ausführungsbeispiel in Form von Klemmelementen 54 und 56 ausgebildet, welche jeweils eine Klemmfläche 58 beziehungsweise 60 aufweisen, die in der Neutralstellung wie in Figur 2 dargestellt aneinander anliegen, jedoch ohne eine Kraft aufeinander auszuüben.

Das vorspannende Element 38 ist so zwischen den Branchen 12 und 14 angeordnet, dass es eine Grundstellung einnimmt. Figur 2 zeigt also das vorspannende Element 38 in seiner Grundstellung mit einer Grundstellungform. Diese ist in einer Draufsicht auf das Instrument 10 kreisförmig.

Das vorspannende Element 38 ist an den Branchen 12 und 14 positionsveränderbar angeordnet, nämlich verschiebbar. Das vorspannende Element 38 kann sowohl in Richtung auf die distalen Enden 16, 18 hin als auch in Richtung auf die proximalen Enden 20, 22 hin verschoben werden.

Figur 3 zeigt schematisch das Instrument 10 mit in proximaler Richtung verschobenem vorspannenden Element 38, also von der Schwenkachse 24 weg in seiner Position verändert. Die Haltepunkte 40 und 42 liegen nun näher an den proximalen Enden 20, 22 als in der Neutralstellung. Da die Werkzeugelemente 32 und 34 bereits aneinander anliegen, können die Branchen 12 und 14 nicht weiter aufeinander zu bewegt werden. Dies hat zur Folge, dass das vorspannende Element 38 durch die Verschiebung in proximaler Richtung verformt wird. Es nimmt nun wie in Figur 3 schematisch dargestellt eine ovale Form ein. Durch die Verformung des vorspannenden Elements aus seiner Grundstellung heraus, nimmt es nun eine Zugstellung ein, in welcher es eine Zugkraft auf die zwei Branchen 12 und 14 aufeinander zu ausübt. Dies führt dazu, dass die Werkzeugelemente 32 und 34 nun nicht mehr ohne eine Kraft aufeinander auszuüben aneinander anliegen, sondern unter Vorspannung gegeneinander gehalten werden. Dies wird durch die beiden Pfeile 62 und 64 schematisch symbolisiert.

Mit anderen Worten ist das Instrument 10, so wie es in Figur 3 schematisch dargestellt ist, ausgehend von der Neutralstellung, wie sie in Figur 2 dargestellt ist, in eine Klemmstellung überführt, in welcher die zusammenwirkenden Werkzeugelemente 32, 34 durch das vorspannende Element unter Vorspannung gegeneinander gehalten sind.

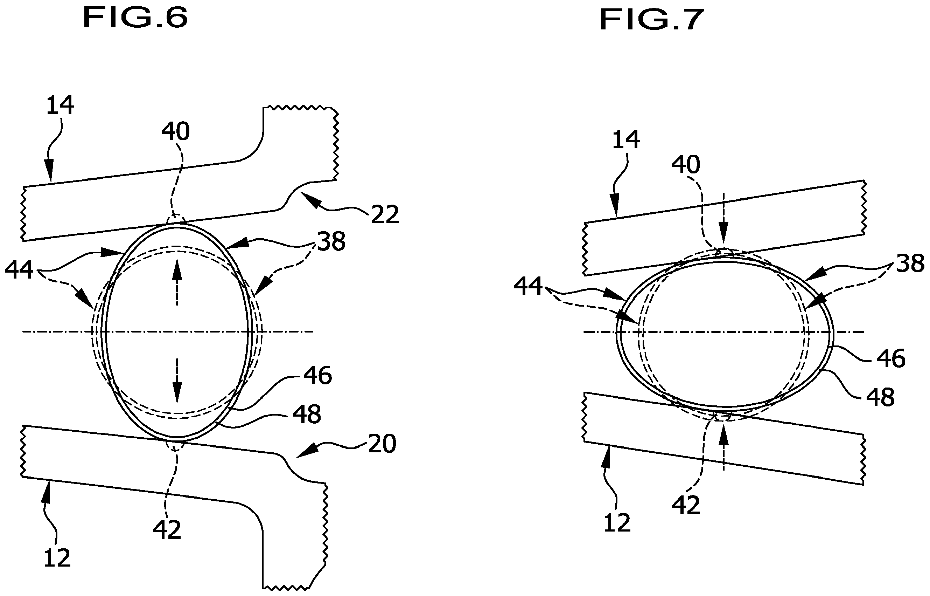

Figur 6 zeigt schematisch die Verformung des vorspannenden Elements 38 in der Klemmstellung im Vergleich zur Neutralstellung. Das vorspannende Element 38 ist in Figur 6 zusätzlich auch in seiner Grundstellungsform schematisch dargestellt, in welcher keine äußeren Verformungskräfte auf das vorspannende Element einwirken. Das in Figur 6 gestrichelt eingezeichnete vorspannende Element 38 ist nimmt die Grundstellung ein, weist also die Grundstellungsform auf, und ist von den zwei Branchen 12, 14 getrennt.

In der Klemmstellung des Instruments 10, wie schematisch in Figur 3 dargestellt, können die zusammenwirkenden Werkzeugelemente 32 und 34 entgegen der Wirkung des vorspannenden Elements 38 voneinander weg bewegt werden. Das vorspannende Element 38 übt dann eine zunehmende Zugkraft auf die Branchen 12, 14 aus.

Aber auch in der Neutralstellung können die zusammenwirkenden Werkzeugelemente 32, 34 entgegen der Wirkung des vorspannenden Elements 38 voneinander weg bewegt werden. Das zunächst keine Kraft ausübende vorspannende Element 38 übt beim Öffnen des Instruments 10, also beim Bewegen der Branchen 12, 14 voneinander weg, eine Zugkraft auf diese aus, um sie wieder in die Neutralstellung zurückzubewegen.

Das vorspannende Element 38 kann jedoch auch ausgehend von der in Figur 2 dargestellten Neutralstellung in Richtung auf die distalen Enden 16, 18 hin, also auch in Richtung auf die Schwenkachse 24 hin, in seiner Position verändert werden. Die Haltepunkte 40 und 42 liegen dann näher bei der Schwenkachse 24 als in der Neutralstellung.

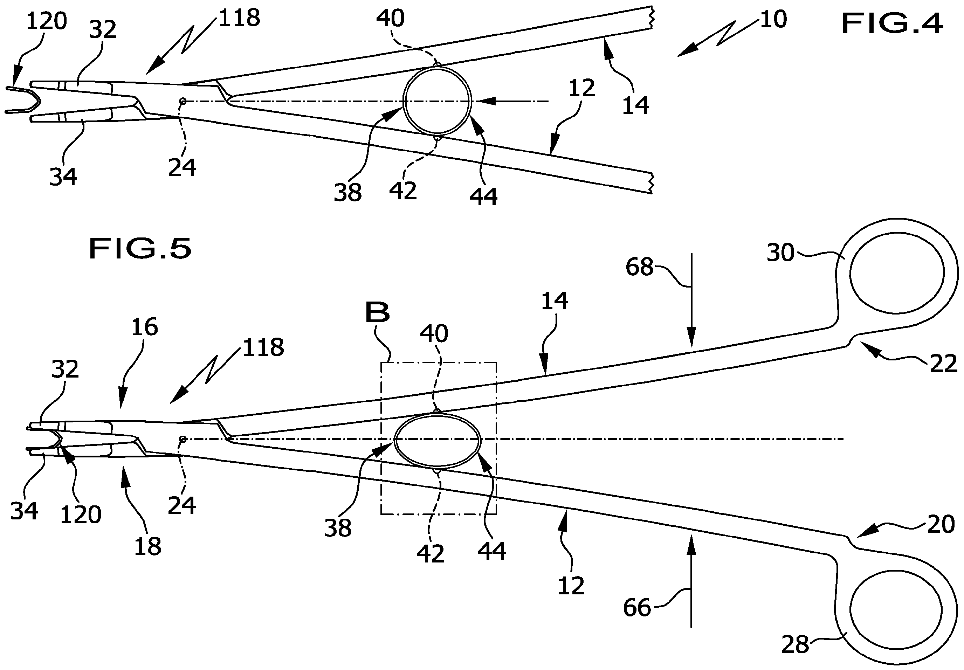

Figur 4 zeigt schematisch eine Aufnahmestellung, in welcher die zusammenwirkenden Werkzeugelemente 32 und 43 durch das vorspannende Element 38 voneinander weg bewegt sind, nämlich voneinander weg verschwenkt. So kann mit dem Instrument 10 beispielsweise ein zu klemmendes Gut, insbesondere Körpergewebe oder ein Tuch, oder ein medizinischer Clip 120 gefasst werden. Beim Übergang von der Neutralstellung in die Aufnahmestellung behält das vorspannende Element 38 die Grundstellung bei beziehungsweise im Wesentlichen bei.

In der Aufnahmestellung können die Branchen 12 und 14, wie dies schematisch durch die Pfeile 66 und 68 dargestellt ist, aufeinander zu bewegt werden. Dabei wird das vorspannende Element 38 zusammengedrückt und übt somit eine Druckkraft auf die Branchen 12 und 14 aus. Mit anderen Worten können die Branchen 12 und 14 in der Aufnahmestellung entgegen der Wirkung des vorspannenden Elements 38 aufeinander zubewegt werden. Das wie in Figur 5 schematisch dargestellt verformte vorspannende Element 38 definiert eine Druckstellung, in welcher es eine Druckkraft auf die zwei Branchen 12 und 14 ausübt. In Figur 7 ist dies nochmals vergrößert dargestellt. Zum Vergleich ist auch hier wie in Figur 6 das vorspannende Element 38 gestrichelt mit seiner Grundstellungsform in der Grundstellung dargestellt.

Das im Zusammenhang mit den Figuren 1 bis 7 beschriebene Ausführungsbeispiel des medizinischen Instruments ist somit derart ausgebildet, dass das vorspannende Element 38 von der Zugstellung, in welcher es eine Zugkraft auf die Branchen 12 und 14 ausübt, in eine Druckstellung bringbar ist, in welcher es eine Druckkraft auf die zwei Branchen 12, 14 ausübt, und zwar durch Änderung seiner Position relativ zu den zwei Branchen 12 und 14.

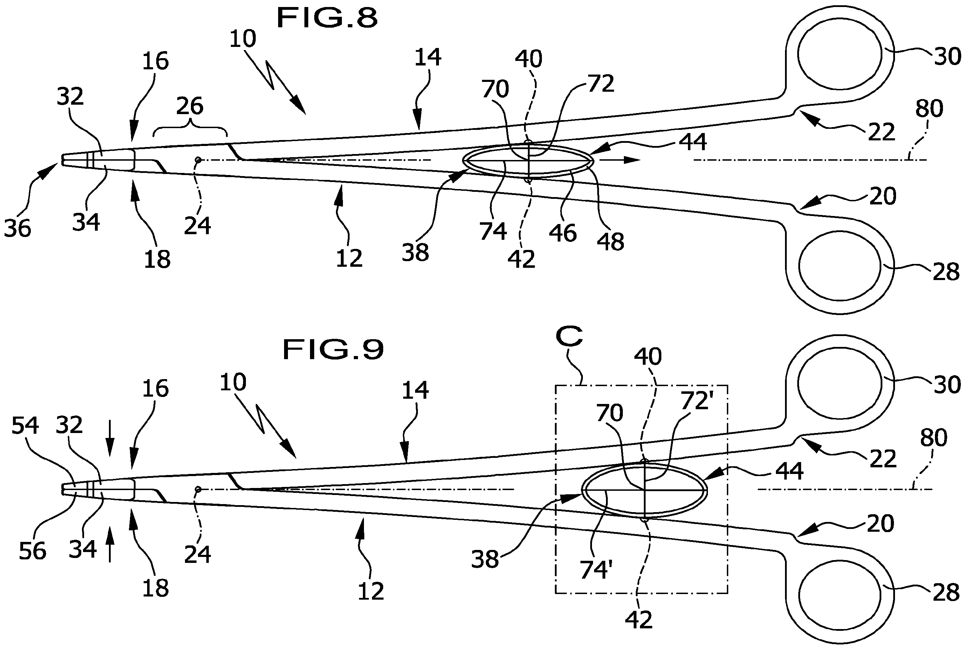

In den Figuren 8 bis 13 ist schematisch ein zweites Ausführungsbeispiel eines medizinischen Instruments 10 dargestellt, welches ein vorspannendes Element 38 umfasst, das von der Zugstellung in die Druckstellung und umgekehrt durch Änderung seiner Orientierung relativ zu den zwei Branchen 12 und 14 bringbar ist. Dies wird nachfolgend im Einzelnen beschrieben. Zur Bezeichnung dieses Ausführungsbeispiels des Instruments 10 werden für identische beziehungsweise in ihrer Funktion äquivalente Komponenten dieselben Bezugszeichen verwendet.

In Figur 8 ist die Neutralstellung des Instruments 10 schematisch dargestellt. Das vorspannende Element 38 nimmt die Grundstellung mit seiner Grundstellungform ein. Die Werkzeugelemente 32 und 34 liegen aneinander an, ohne eine Kraft aufeinander auszuüben.

Das vorspannende Element 38 definiert bei diesem Ausführungsbeispiel eine Längsachse 70. Das vorspannende Element 38 weist eine rotationsunsymmetrische Grundstellungsform auf. Es weist in der Draufsicht in Figur 8 eine ovale Form mit einer ersten Ausdehnung 72 und einer zweiten Ausdehnung 74. Die Ausdehnungen 72 und 74 erstrecken sich in der Grundstellung in zwei voneinander linear unabhängigen Richtungen quer, nämlich senkrecht zueinander. Die erste Ausdehnung 72 ist kleiner als die zweite Ausdehnung 74. In der in Figur 8 schematisch dargestellten Neutralstellung ist das vorspannende Element 38 in der Grundstellung in Richtung der ersten Ausdehnung 72 an den Haltepunkten 40 und 42 der Branchen 12 und 14 gehalten.

Das Instrument 10 ist in die Klemmstellung überführbar durch Ändern der Position des vorspannenden Elements 38 an den Branchen 12 und 14, und zwar durch Verschieben in proximaler Richtung. In Figur 9 sind die erste Ausdehnung und die zweite Ausdehnung mit dem Bezugszeichen 72' und 74' bezeichnet. Die Ausdehnung 72' ist etwas größer als die Ausdehnung 72 in der Grundstellung, und die Ausdehnung 74' ist etwas kleiner als die Ausdehnung 74 in der Grundstellung. Das vorspannende Element 38 ist aus seiner Grundstellung heraus verformt und übt eine Zugkraft auf die Branchen 12 und 14 aufeinander zu aus. Dadurch werden die Klemmelemente 32 und 34 gegeneinander gedrückt. Das Instrument 10 nimmt also die Klemmstellung ein.

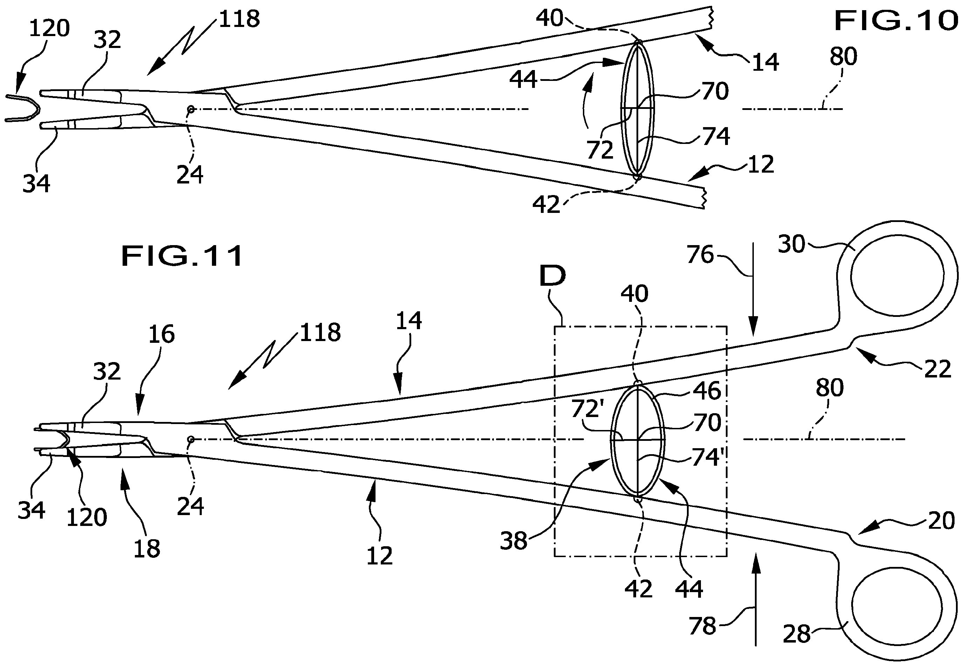

Wahlweise ausgehend von der Neutralstellung, wie sie schematisch in Figur 8 dargestellt ist, oder ausgehend von der Klemmstellung, wie sie schematisch in Figur 9 dargestellt ist, kann das vorspannende Element 38 in seiner Orientierung geändert werden, und zwar durch Drehung um die Längsachse 70. Figur 10 zeigt dies schematisch ausgehend von der Neutralstellung. Durch die Änderung der Orientierung erstreckt sich nun das vorspannende Element 38 mit der zweiten Ausdehnung 74 zwischen den Haltepunkten 40 und 42 an den Branchen 12 und 14.

Durch die Drehung des vorspannenden Elements 38 werden die Branchen 12 und 14 voneinander weg bewegt und die Werkzeugelemente 32 und 34 voneinander weg verschwenkt. Das vorspannende Element 38 nimmt nach wie vor die Grundstellung ein und hält das Instrument 10 kraftneutral in einer geöffneten Stellung.

Werden die Branchen 12 und 14 ausgehend von der in Figur 10 dargestellten Aufnahmestellung in Richtung der Pfeile 76 und 78 aufeinander zu bewegt, wird das vorspannende Element 38 verformt und nimmt dann die Druckstellung ein, in welcher es eine Druckkraft auf die Branchen 12 und 14 ausübt, die dem Schließen des Instruments 10 entgegenwirkt. Werden die Branchen 12 und 14 losgelassen, also die Beaufschlagung mit Kräften in Richtung der Pfeile 76 und 78 unterbrochen, drückt das vorspannende Element 38 das Instrument 10 durch Übergang von der Druckstellung in die Grundstellung in eine weiter geöffnete Aufnahmestellung zurück.

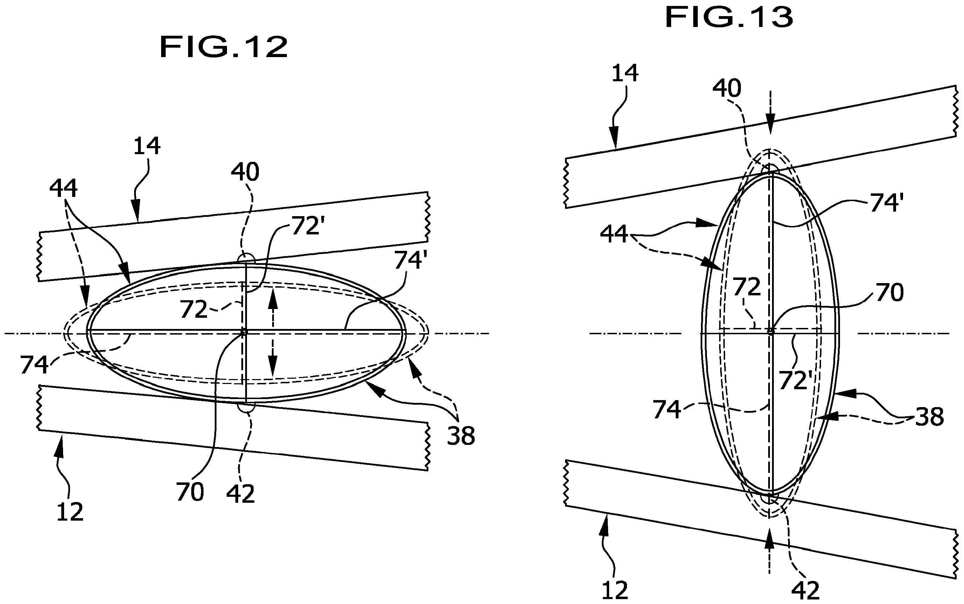

Figur 12 zeigt schematisch den vergrößerten Ausschnitt C in Figur 9. Das vorspannende Element 38 ist aus der Grundstellung heraus verformt und nimmt die Zugstellung ein, in welcher es eine Zugkraft auf die Branchen 12 und 14 zum Bewegen derselben aufeinander zu ausübt. Durch die Änderung der Position ausgehend von der Neutralstellung in Figur 8 in die Klemmstellung in Figur 9 vergrößert sich die erste Ausdehnung 72 zur ersten Ausdehnung 72' und verringert sich die zweite Ausdehnung 74 zur zweiten Ausdehnung 74'.

In Figur 13 ist schematisch der Bereich D aus Figur 11 vergrößert dargestellt. Die Branchen 12 und 14 sind hier bereits etwas gegeneinander bewegt, sodass das vorspannende Element 38 die Druckstellung einnimmt. Im Vergleich zur Druckstellung ist das vorspannende Element 38 zusätzlich in der Grundstellung mit seiner Grundstellungsform gestrichelt eingezeichnet.

Ergänzend sei angemerkt, dass das vorspannende Element 38 auch ausgehend von der in Figur 9 dargestellten Klemmstellung des Instruments 10 um die Schwenkachse 70 gedreht werden kann. Dabei geht es von der Zugstellung, über die Neutralstellung in die in Figur 10 beziehungsweise in Figur 11 dargestellte Druckstellung über. Die Neutralstellung ist dabei eine Stellung, in welcher das vorspannende Element 38 schräg bezogen auf eine Längsachse 80 des Instruments orientiert ist. Schräg bedeutet hier, dass weder die erste Ausdehnung 72 noch die zweite Ausdehnung 74 parallel zur Längsachse 80 des Instruments ausgerichtet ist. Die Längsachse 80 des Instruments verläuft quer zur Schwenkachse 24 und erstreckt sich zwischen den Branchen 12 und 14.

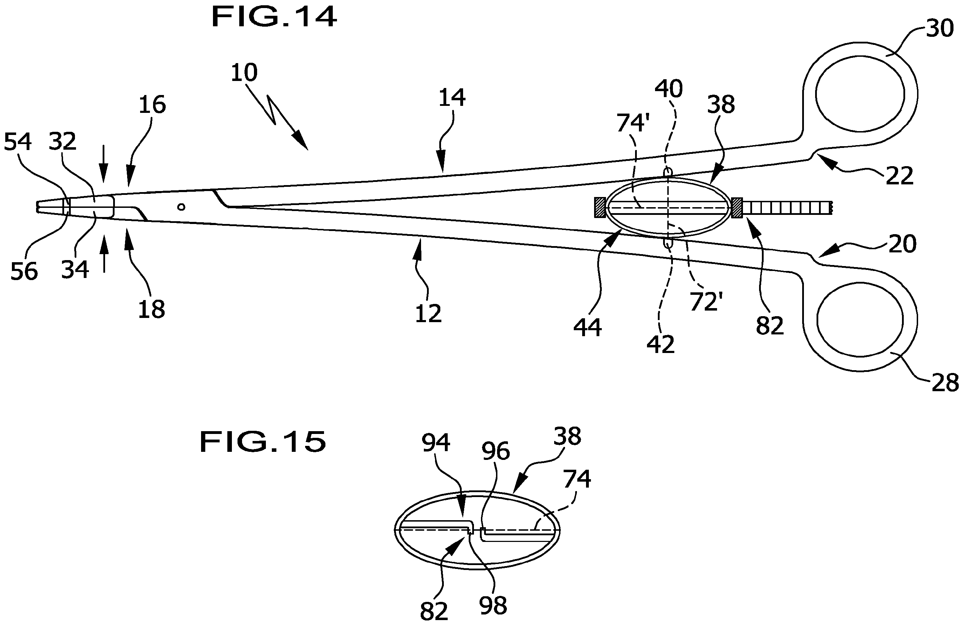

In den Figuren 14, 16 und 17 ist ein weiteres Ausführungsbeispiel eines medizinischen Instruments 10 schematisch dargestellt. Es unterscheidet sich von den Instrumenten 10 der Figuren 1 bis 13 lediglich durch die Ausgestaltung des vorspannenden Elements 38.

In Figur 14 ist das Instrument 10 in der Klemmstellung dargestellt. Das vorspannende Element 38 ist hier, ähnlich wie bei dem Ausführungsbeispiel der Figur 9, aus seiner Grundstellung heraus etwas verformt, mit einer ersten Ausdehnung 72', die etwas größer ist als die Ausdehnung 72 in der Grundstellung. Entsprechend ist die zweite Ausdehnung 74' etwas kleiner als die Ausdehnung 74 in der Grundstellung des vorspannenden Elements 38.

Bei diesem Ausführungsbeispiel des medizinischen Instruments 10 wird das vorspannende Element 38 von der Zugstellung in die Druckstellung gebracht und umgekehrt durch Änderung seiner Form relativ zu den zwei Branchen. Hierfür umfasst das Instrument 10 eine Formänderungseinrichtung 82, um die Form des vorspannenden Elements 38 zu ändern. Die Formänderungseinrichtung 82 ist mit dem vorspannenden Element 38 zusammenwirkend angeordnet beziehungsweise ausgebildet, um die erste Ausdehnung 72 zu vergrößern sowie die zweite Ausdehnung 74 zu verringern.

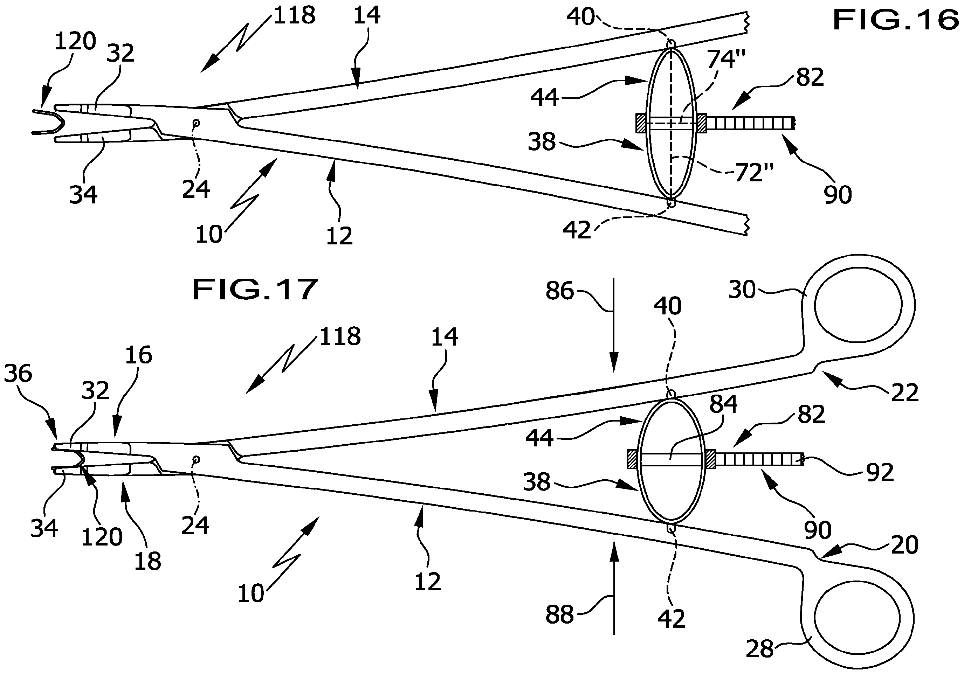

Bei dem Ausführungsbeispiel der Figuren 14, 16 und 17 umfasst die Formänderungseinrichtung 82 ein in seiner Länge veränderbares Band, welches das vorspannende Element 38 in der Grundstellung in Richtung der zweiten Ausdehnung 74 umgibt. Durch Verkürzen des Bands 84 kann das Instrument 10 von der Neutralstellung beziehungsweise der Klemmstellung in die Aufnahmestellung überführt werden. Dies ist in Figur 16 schematisch dargestellt. Hier ist eine Länge des Bands 84 in Richtung der zweiten Ausdehnung 74 so verkürzt, dass die zweite Ausdehnung 74" sehr viel geringer ist als die Ausdehnung 74' in der Klemmstellung. Dagegen ist die erste Ausdehnung 72" hier signifikant größer als die erste Ausdehnung 72' in der Klemmstellung.

Ausgehend von der geänderten Form des vorspannenden Elements 38 kann das Instrument 10, und zwar durch Ausüben von Kräften auf die Branchen 12 und 14 aufeinander zu, was durch die Pfeile 86 und 88 symbolisiert ist, wiederum das vorspannende Element 38 in seiner Form ändern, sodass es eine Druckkraft auf die Branchen 12 und 14 ausübt, um diese wieder voneinander weg zu bewegen.

Bei dem Ausführungsbeispiel der Figuren 14, 16 und 17 ist das Band 84 in Form eines Kabelbinders 90 ausgebildet. Wird am freien Ende 92 des Kabelbinders 90 gezogen, verringert sich eine freie Länge des Bands 84 in Richtung der zweiten Ausdehnung 74 und verformt dabei das vorspannende Element 38 wie beschrieben.

Figur 15 zeigt schematisch ein weiteres Ausführungsbeispiel eines vorspannenden Elements 38 in der Grundstellung. In der Draufsicht weist es wiederum eine ovale Form auf, ähnlich wie das vorspannende Element 38 in Figur 14. Auch dieses Ausführungsbeispiel eines vorspannenden Elements 38 wirkt mit einer Formänderungseinrichtung 82 zusammen. Diese ist in Form einer Sperre 94 ausgebildet, welche in Richtung der zweiten Ausdehnung 74 wirkt. Die Sperre 94 umfasst zwei zusammenwirkende, aufeinander zu weisende Sperrhaken 96 und 98, die in der Grundstellung des vorspannenden Elements 38 wie in Figur 5 außer Eingriff stehen. Das in Figur 15 mit einer derart ausgestalteten Sperre 94 schematisch dargestellte Ausführungsbeispiel des vorspannenden Elements 38 kann das vorspannende Element 38, wie es in Figur 14 dargestellt ist, ersetzen.

Um die zweite Ausdehnung 74 signifikant zu verringern, können die Sperrhaken 96 und 98 miteinander in Eingriff gebracht werden, sodass sich eine ähnliche Verformung ergibt, wie sie schematisch in Figur 16 für die Formänderungseinrichtung 82 umfassend das Band 84 schematisch dargestellt ist.

Der Kabelbinder 90 ermöglicht eine im Wesentlichen stufenlose Änderung der Ausdehnung 72 und 74. Durch die Sperre 94 lassen sich die Ausdehnungen 72, 74 in einer Stufe ändern.

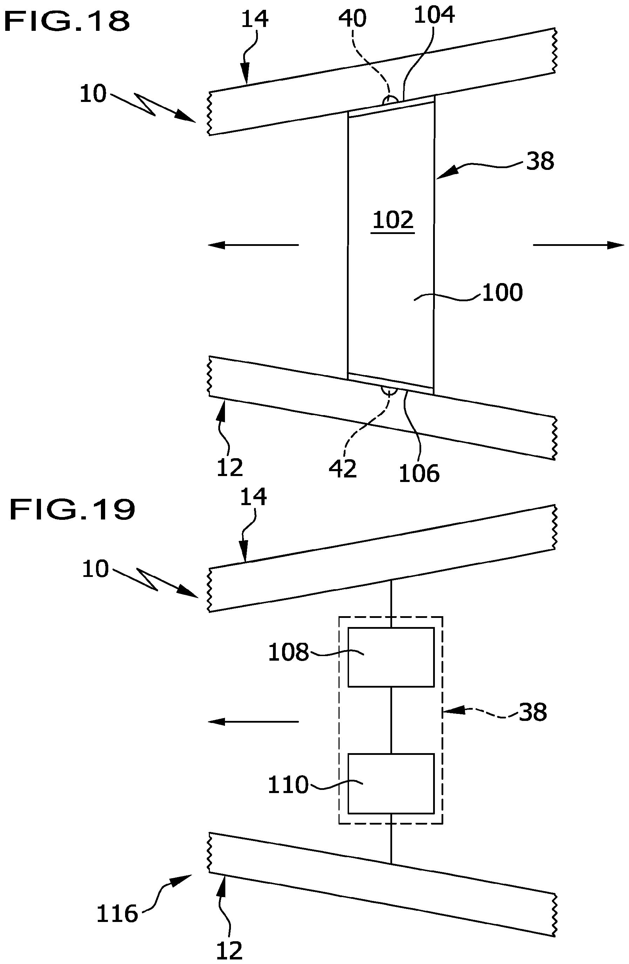

Figur 18 zeigt schematisch einen Ausschnitt eines weiteren Ausführungsbeispiels eines Instruments 10 ähnlich der Figur 6. Das vorspannende Element 38 ist bei diesem Ausführungsbeispiel in Form eines gummielastischen Elements 100. Das gummielastische Element 100 weist bei diesem Ausführungsbeispiel die Form eines stabförmigen Gummikörpers 102 auf. Voneinander weg weisende Enden 104 und 106 des Gummikörpers 102 sind an den Haltepunkten 40 und 42 an den zwei Branchen 12 und 14 angeordnet beziehungsweise gehalten.

Der Gummikörper 102 ist wie das vorspannende Element 38 beim Ausführungsbeispiel der Figuren 1 bis 7 positionsveränderbar an den Branchen 12 und 14 angeordnet, kann also in proximaler beziehungsweise distaler Richtung an den Branchen 12 und 14 unterschiedlich positioniert, insbesondere verschoben, werden, sodass das vorspannende Element 38 ausgehend von seiner Grundstellung in die Druckstellung einerseits und in die Zugstellung andererseits überführbar ist. So kann das Instrument 10 mit dem Gummikörper 102 in analoger Weise wie das Instrument 10 gemäß dem Ausführungsbeispiel der Figuren 1 bis 7 von einer Neutralstellung in eine Klemmstellung sowie in eine Aufnahmestellung überführt werden.

Figur 19 zeigt schematisch ein Ersatzschaltbild des vorspannenden Elements 38. Es umfasst ein Druckelement 108 und ein Zugelement 110. Das Druckelement 108 ist komprimierbar ausgebildet, um eine entsprechende Druckkraft auszuüben. Das Zugelement 110 ist auseinanderziehbar, um eine entsprechende Zugkraft auszuüben. Diese schematische Darstellung zeigt, wie Eigenschaften des vorspannenden Elements 38 prinzipiell gezielt geändert werden können. Insbesondere können durch Trennung von Druck- und Zugeigenschaften des vorspannenden Elements unterschiedliche Charakteristika beim Öffnen und Schließen des Instruments 10 eingestellt werden. Dieses Ausführungsbeispiel eines Instruments 10 ist schematisch als Nadelhalter 116 dargestellt.

Die beschriebenen Ausführungsbeispiele vorspannender Elemente 38 können insbesondere aus einem Metall, beispielsweise aus einem Federstahl, oder aus einem Kunststoff ausgebildet sein. Insbesondere die Ringfeder 46 kann wahlweise aus einem Metall oder einem Kunststoff ausgebildet sein.

Die medizinische Werkzeugeinrichtung 36 kann zur Ausbildung unterschiedlicher Instrumente 10 beispielsweise in Form einer Schneideinrichtung oder in Form einer Klemmeinrichtung 112 wie schematisch bei den Ausführungsbeispielen der Figuren 1 bis 14 dargestellt, ausgebildet sein. Eine Schneideinrichtung kann ausgebildet werden durch Werkzeugelemente 32 und 34, die in Form von zusammenwirkenden Schneiden ausgebildet sind.

Die in den Figuren dargestellten Ausführungsbeispiele medizinischer Instrumente 10 sind in Form von Ringinstrumenten 114 ausgebildet. Alternativ kann auf die Fingerringe 28 und 30 auch verzichtet werden.

Durch entsprechende Gestaltung der Werkzeugelemente 32, 34 kann das medizinische Instrument 10 beispielsweise in Form eines Nadelhalters, einer Schere oder einer medizinischen Klemme, beispielsweise einer Tuchklemme ausgebildet werden. Die Ausführungsbeispiele der Figuren 1 bis 17 zeigen schematisch Clippanlegeinstrumente 118, mit welchen medizinische Clips 120 appliziert werden können.

Bei einem weiteren Ausführungsbeispiel ist das medizinische Instrument 10 modular ausgebildet. Es umfasst zwei sich in Form beziehungsweise Größe beziehungsweise seiner vorspannenden Charakteristik unterscheidende vorspannende Elemente 38, die wahlweise mit den zwei Branchen 12 und 14 koppelbar sind. Diese Ausgestaltung ermöglicht es insbesondere, ein medizinisches Instrumentensystem bereitzustellen, welches für einen Anwender individualisierbar ist. Je nach Größe und vorspannender Kraft, die das vorspannende Element 38 ausüben kann, kann so eine Öffnungsweite und auch eine Schließ- oder Betätigungskraft des Instruments 10 für einen Anwender vorgegeben werden, und zwar je nach dessen Vorlieben.

Die Positionsveränderbarkeit des vorspannenden Elements 38 an den Branchen 12 und 14 kann beispielsweise stufenlos vorgesehen sein oder auch in diskreten Stufen. Dis lässt sich insbesondere über die Art der Befestigung des vorspannenden Elements 38 an den Branchen 12 und 14 erreichen. Beispielsweise können die Haltepunkte 40 und 42, an denen das vorspannende Element 38 gehalten ist, an den Branchen 12 und 14 verschiebbar angeordnet beziehungsweise ausgebildet sein. Alternativ können auch an den Branchen 12 und 14 eine Mehrzahl von Haltepunkten 40 und 42 angeordnet beziehungsweise ausgebildet sein, um diskrete Positionen für das vorspannende Element 38 vorzugeben. Insbesondere kann es dann auch in unterschiedlichen Orientierungen an den Haltepunkten 40 und 42 gekoppelt werden.

Die beschriebenen Ausführungsbeispiele medizinischer Instrumente 10 weisen insbesondere gute Handhabungseigenschaften auf. Zudem ermöglichen sie es, auf eine Sperre zu verzichten, denn wenn das vorspannende Element 38 so positioniert oder orientiert oder in seiner Form geändert ist, dass das Instrument 10 die Klemmstellung einnimmt, wird eine Sperreinrichtung, wie sie bei herkömmlichen Instrumenten häufig eingesetzt wird, überflüssig. Zudem sind aufgrund der jeweiligen Ausgestaltung der vorspannenden Elemente 38 die beschriebenen Instrumente 10 spielfrei. Sie weisen nicht das bekannte und für einen Anwender unangenehme, eingangs beschriebene Blattfedersteckspiel auf. Die Stellung der Branchen 12 und 14 relativ zueinander ist bei den beschriebenen Ausführungsbeispielen durch das vorspannende Element 38 definiert und spielfrei vorgegeben. Es gibt also keine "labile" Situation wie bei herkömmlichen Instrumenten. Somit kann bei den wie vorgeschlagen verbesserten Instrumenten 10 ein Anwender beim Aufgreifen eines solchen Instruments 10 auch nicht gefühlt ins Leere greifen. Die Instrumente weisen somit eine deutlich verbesserte Haptik auf.

### Bezugszeichenliste

- 10: Instrument
- 12: erste Branche
- 14: zweite Branche
- 16: distales Ende
- 18: distales Ende
- 20: proximales Ende
- 22: proximales Ende
- 24: Schwenkachse
- 26: Schlussbereich
- 28: Fingerring
- 30: Fingerring
- 32: Werkzeugelement
- 34: Werkzeugelement
- 36: Werkzeugeinrichtung
- 38: vorspannendes Element
- 40: Haltepunkt
- 42: Haltepunkt
- 44: federelastisches Element
- 46: Ringfeder
- 48: Federband
- 50: Breite
- 52: Dicke
- 54: Klemmelement
- 56: Klemmelement
- 58: Klemmfläche
- 60: Klemmfläche
- 62: Pfeil
- 64: Pfeil
- 66: Pfeil
- 68: Pfeil
- 70: Längsachse
- 72: erste Ausdehnung
- 74: zweite Ausdehnung
- 76: Pfeil
- 78: Pfeil
- 80: Längsachse
- 82: Formänderungseinrichtung
- 84: Band
- 86: Pfeil
- 88: Pfeil
- 90: Kabelbinder
- 92: Ende
- 94: Sperre
- 96: Sperrhaken
- 98: Sperrhaken
- 100: gummielastische Element
- 102: Gummikörper
- 104: Ende
- 106: Ende
- 108: Druckelement
- 110: Zugelement
- 112: Klemmeinrichtung
- 114: Ringinstrument
- 116: Nadelhalter
- 118: Clipanlegeinstrumet
- 120: Clip

## Patentansprüche

1. Medizinisches Instrument (10) mit zwei relativ zueinander bewegbaren Branchen (12, 14), wobei jede Branche (12, 14) ein distales Ende (16, 18) mit einem Werkzeugelement (32, 34) und ein proximales Ende (20, 22) aufweist und wobei die Werkzeugelemente (32, 34) miteinander zusammenwirkend angeordnet und ausgebildet sind und eine medizinische Werkzeugeinrichtung (36) bilden, wobei das medizinische Instrument (10) ein vorspannendes Element (38) umfasst, welches mit den zwei Branchen (12, 14) zusammenwirkend angeordnet oder ausgebildet ist, **dadurch gekennzeichnet, dass** das vorspannende Element (38) von einer Zugstellung, in welcher es eine Zugkraft auf die zwei Branchen (12, 14) ausübt, in eine Druckstellung, in welcher es eine Druckkraft auf die zwei Branchen (12, 14) ausübt, bringbar ist oder umgekehrt durch Änderung seiner Position und/oder seiner Orientierung und/oder seiner Form relativ zu den zwei Branchen (12, 14).

2. Medizinisches Instrument nach Anspruch 1, **dadurch gekennzeichnet, dass** das vorspannende Element (38) an der ersten Branche (12) und der zweiten Branche (14) gehalten ist.

3. Medizinisches Instrument nach einem der voranstehenden Ansprüche, **dadurch gekennzeichnet, dass** die zwei Branchen (12, 14) in einem Schlussbereich (26) um eine Schwenkachse (24) verschwenkbar aneinander gelagert sind und dass das vorspannende Element (38) zwischen dem Schlussbereich (26) und den proximalen Enden (20, 22) mit den zwei Branchen (12, 14) zusammenwirkt.

4. Medizinisches Instrument nach einem der voranstehenden Ansprüche, **dadurch gekennzeichnet, dass** das vorspannende Element (38) an den Branchen (12, 14) positionsveränderbar, insbesondere verschiebbar, angeordnet ist, insbesondere sowohl in Richtung auf die distalen Enden (16, 18) hin als auch in Richtung auf die proximalen Enden (20, 22) der zwei Branchen hin (12, 14),
wobei insbesondere das vorspannende Element (38) an den zwei Branchen (12, 14)
a) in Richtung auf die Schwenkachse (24) hin und von der Schwenkachse (24) weg positionsveränderbar ist
und/oder
b) stufenlos positionsveränderbar oder in diskreten Positionen mit den zwei Branchen (12, 14) zusammenwirkend anordenbar ist.

5. Medizinisches Instrument nach einem der voranstehenden Ansprüche, **dadurch gekennzeichnet, dass** das vorspannende Element (38) eine Grundstellung definiert, in welcher die zwei Branchen (12, 14) keine Kraft auf das vorspannende Element (38) ausüben und in welcher das vorspannende Element (38) keine Kraft auf die zwei Branchen (12, 14) ausübt,
wobei insbesondere
a) das vorspannende Element (38) in der Grundstellung eine Grundstellungsform aufweist
und/oder
b) in der Grundstellung keine äußeren Verformungskräfte auf das vorspannende Element (38) einwirken
und/oder
c) das von mindestens einer der zwei Branchen (12, 14) getrennte vorspannende Element (38) die Grundstellung definiert.

6. Medizinisches Instrument nach Anspruch 5, **dadurch gekennzeichnet, dass** in einer Neutralstellung des Instruments (10) die zwei Werkzeugelemente (32, 34) aneinander anliegen, ohne eine Kraft aufeinander auszuüben.

7. Medizinisches Instrument nach Anspruch 6, **dadurch gekennzeichnet, dass**
a) das vorspannende Element (38) in der Neutralstellung des Instruments (10) die Grundstellung einnimmt
und/oder
b) die zusammenwirkenden Werkzeugelemente (32, 34) ausgehend von der Neutralstellung entgegen der Wirkung des vorspannenden Elements (38) voneinander weg bewegbar sind.

8. Medizinisches Instrument nach Anspruch 6 oder 7, **dadurch gekennzeichnet, dass** das Instrument von der Neutralstellung in eine Aufnahmestellung, in welcher die zusammenwirkenden Werkzeugelement (32, 34) durch das vorspannende Element (38) voneinander weg bewegt sind, insbesondere voneinander weg verschwenkt, bringbar ist durch Änderung seiner Position und/oder seiner Orientierung und/oder seiner Form relativ zu den zwei Branchen,
wobei insbesondere
a) das vorspannende Element (38) beim Übergang des Instruments (10) von der Neutralstellung in die Aufnahmestellung die Grundstellung beibehält
und/oder
b) die zusammenwirkenden Werkzeugelemente (32, 34) in der Aufnahmestellung entgegen der Wirkung des vorspannenden Elements (38) aufeinander zu bewegbar sind.

9. Medizinisches Instrument nach einem der Ansprüche 6 bis 8, **dadurch gekennzeichnet, dass** das Instrument von der Neutralstellung in eine Klemmstellung, in welcher die zusammenwirkenden Werkzeugelemente (32, 34) durch das vorspannende Element (38) unter Vorspannung gegeneinander gehalten sind, bringbar ist durch Überführen des vorspannenden Elements (38) von der Neutralstellung in die Zugstellung durch Änderung seiner Position und/oder seiner Orientierung und/oder seiner Form relativ zu den zwei Branchen (12, 14).

10. Medizinisches Instrument nach einem der Ansprüche 6 bis 9, **dadurch gekennzeichnet, dass** das vorspannende Element (38) eine Längsachse (70) definiert und dass es in der Grundstellung in zwei voneinander linear unabhängigen Richtungen, welche quer, insbesondere senkrecht, zur Längsachse orientiert sind, eine erste Ausdehnung (72) und eine zweite Ausdehnung (74) aufweist, dass die erste Ausdehnung (72) kleiner ist als die zweite Ausdehnung (74) und dass in der Neutralstellung oder in der Klemmstellung das vorspannende Element (38) mit den zwei Branchen (12, 14) in Richtung der ersten Ausdehnung (72) an den zwei Branchen (12, 14) gehalten ist.

11. Medizinisches Instrument nach Anspruch 10, **dadurch gekennzeichnet, dass** das Instrument (10) durch Änderung
a) der Orientierung des vorspannenden Elements (38), nämlich insbesondere durch Drehung um die Längsachse (70), von der Neutralstellung in die Aufnahmestellung und umgekehrt oder von der Neutralstellung in der Klemmstellung und umgekehrt bringbar ist
und/oder
b) der Form des vorspannenden Elements (38), nämlich insbesondere durch Vergrößern der ersten Ausdehnung (72) und/oder Verringern der zweiten Ausdehnung (74), von der Neutralstellung in die Aufnahmestellung und umgekehrt oder von der Neutralstellung in die Klemmstellung, nämlich insbesondere durch Verringern der ersten Ausdehnung (72) und/oder Vergrößern der zweiten Ausdehnung (74), und umgekehrt bringbar ist.

12. Medizinisches Instrument nach einem der voranstehenden Ansprüche, **dadurch gekennzeichnet, dass** das Instrument (10) eine Formänderungseinrichtung (82) umfasst zum Ändern der Form des vorspannenden Elements (38).

13. Medizinisches Instrument nach Anspruch 12, **dadurch gekennzeichnet, dass**, die Formänderungseinrichtung (82)
a) mit dem vorspannenden Element (38) zusammenwirkend angeordnet oder ausgebildet ist zum Vergrößern der ersten Ausdehnung (72) und/oder Verringern der zweiten Ausdehnung (74)
und/oder
b) in Form einer in Richtung der ersten Ausdehnung (72) oder der zweiten Ausdehnung (74) wirkenden Sperre (94) ausgebildet ist, insbesondere umfassend zwei zusammenwirkende, aufeinander zu weisende Sperrhaken (96, 98), die in der Neutralstellung und/oder der Klemmstellung außer Eingriff stehen und in der Aufnahmestellung in Eingriff stehen
und/oder
c) ein in seiner Länge veränderbares Band (84) umfasst, welches das vorspannende Element (38) in der Grundstellung in Richtung der zweiten Ausdehnung (74) umgibt, und dass durch Verkürzen des Bands (84) das Instrument (10) von der Neutralstellung und/oder der Klemmstellung in die Aufnahmestellung bringbar ist.

14. Medizinisches Instrument nach einem der voranstehenden Ansprüche, **dadurch gekennzeichnet, dass** das vorspannende Element (38) in Form eines federelastischen Elements (44) und/oder in Form eines gummielastischen Elements (100) ausgebildet ist,
wobei insbesondere
a) das federelastische Element (44) in Form einer Schraubenfeder oder einer in sich geschlossenen Ringfeder (46) ausgebildet ist
b) das gummielastische Element (100) in Form eines, insbesondere stabförmigen, Gummikörpers (102) ausgebildet ist, wobei insbesondere voneinander weg weisende Enden (104, 106) des Gummikörpers (102) jeweils an einer der zwei Branchen (12, 14) angeordnet sind.

15. Medizinisches Instrument nach einem der voranstehenden Ansprüche, **dadurch gekennzeichnet, dass** das medizinische Instrument (10)
a) in Form eines Ringinstruments (114) ausgebildet ist und an den proximalen Enden (20, 22) der zwei Branchen (12, 14) ein Ring (28, 30) angeordnet oder ausgebildet ist
und/oder
b) in Form eines Nadelhalters, einer Schere oder einer medizinischen Klemme, insbesondere einer Tuchklemme, ausgebildet ist
und/oder
c) modular ausgebildet ist und mindestens zwei sich in Form und/oder Größe und/oder in ihrer vorspannenden Charakteristik unterscheidende vorspannende Elemente (38) umfasst, welche wahlweise mit den zwei Branchen (12, 14) koppelbar sind.
